# EUROPÄISCHE PATENTANMELDUNG

(11) **EP 4 674 448 A1**
(43) Veröffentlichungstag der Anmeldung: **07.01.2026**
(21) Anmeldenummer: 24185764.8
(22) Anmeldetag: 01.07.2024
(51) Int. Cl.: A61M 5/142

(54) **INFUSIONSPUMPE MIT AUTHENTIFIZIERUNGSVORRICHTUNG**

(71) Anmelder: B. Braun Melsungen AG, 34212 Melsungen (DE)
(72) Erfinder: Schwarz, Jan, 34212 Melsungen (DE)
(74) Vertreter: Tergau & Walkenhorst Intellectual Property GmbH

(57) **Zusammenfassung**

Infusionspumpe (2), welche zur Verwendung wenigstens eines Infusionsartikels (38) ausgebildet ist, wobei die Infusionspumpe (2) wenigstens eine Authentifizierungsvorrichtung (42) zur Authentifizierung des wenigstens einen Infusionsartikels (38) zur Verwendung mit der Infusionspumpe (2).

## Beschreibung

Die Erfindung betrifft eine Infusionspumpe nach dem Oberbegriff von Anspruch 1. Sie betrifft weiterhin ein Infusionssystem und ein Verfahren zum Betreiben einer Infusionspumpe.

Infusionseinmalartikel sind wesentliche Komponenten im Gesundheitswesen, die in Verbindung mit Infusionspumpen verwendet werden, um die Verabreichung von Flüssigkeiten, Medikamenten und Nährstoffen an Patienten zu gewährleisten. Diese Artikel, die in der Regel sterile Infusionssets, Schläuche und Beutel sowie Spritzen umfassen, sind speziell dafür konzipiert, nur einmal verwendet zu werden, um das Risiko von Kreuzkontaminationen und Infektionen zu minimieren.

Infusionspumpen, die als Peristaltikpumpen oder Spritzenpumpen ausgebildet sein können, dienen dazu, präzise und kontrollierte Mengen an Flüssigkeiten in den Blutkreislauf eines Patienten abgeben. Die Integration von Infusionseinmalartikeln, insbesondere Schläuchen, Beuteln und Spritzen, in diese Pumpen sorgt nicht nur für eine hohe Genauigkeit und Sicherheit bei der Dosierung, sondern auch für eine verbesserte Patientenversorgung und -sicherheit. Die Verwendung dieser Einwegprodukte spielt somit eine zentrale Rolle in der modernen medizinischen Praxis, indem sie eine sterile und effiziente Medikamentenverabreichung ermöglicht.

Aus der WO 2015/150280 A1 ist bekannt, die Konfiguration und den Zustand eines Infusionssystems zu überwachen. Dazu wird mittels eines Signalgebers aktiv in die Flüssigkeit ein Signal eingeleitet, das durch die Flüssigkeit übertragen wird und an anderer Stelle, vom Signalgeber beabstandet, mittels eines Sensors erfasst wird.

Der Erfindung liegt die Aufgabe zugrunde, eine Infusionspumpe bereitzustellen, bei welcher die Nachteile des Standes der Technik vermieden werden. Insbesondere soll nur eine Verwendung von dafür zertifizierten Infusionsartikeln mit der Infusionspumpe ermöglicht werden. Weiterhin sollen ein verbessertes Infusionssystem und ein verbessertes Verfahren zum Betreiben einer Infusionspumpe bereitgestellt werden.

In Bezug auf die Infusionspumpe wird diese Aufgabe erfindungsgemäß gelöst durch eine Infusionspumpe mit den Merkmalen von Anspruch 1. Die Infusionspumpe, welche zur Verwendung wenigstens eines Infusionsartikels bzw. Infusionseinmalartikels ausgebildet ist, weist wenigstens eine Authentifizierungsvorrichtung zur Authentifizierung des wenigstens einen Infusionsartikels auf.

Vorteilhafte Ausgestaltungen der Erfindung sind Gegenstand der Unteransprüche.

Die Erfindung geht von der Überlegung aus, dass es für eine zuverlässige Versorgung von Patienten und die Vermeidung von Kontaminationen wichtig ist, dass der Infusionsartikel für die Infusionspumpe zugelassen zertifiziert ist, da nur so zuverlässig Patientenschaden abgewehrt werden kann. Der Infusionsartikel muss für die Infusionspumpe qualifiziert sein und damit ein authentifizierter Infusionsartikel sein.

Wie nunmehr erkannt wurde, kann zuverlässig Patientenschaden durch nicht geeignete Infusionsartikel verhindert werden, indem der Infusionsartikel vor Inbetriebnahme mit der Infusionspumpe zunächst von dieser authentifiziert wird.

Vorteilhafterweise weist die wenigstens eine Authentifizierungsvorrichtung einen Sensor auf. Mit Hilfe des Sensors kann ein auf dem Infusionsartikel angeordnetes Authentifizierungsmerkmal erfasst werden.

Bevorzugt ist der Sensor ein elektromagnetischer Sensor. Vorteilhafterweise ist der Sensor dabei als optischer Sensor, insbesondere als Kamera, ausgebildet.

Alternativ kann der elektromagnetische Sensor als RFID-Reader oder NFC-Sensor ausgebildet sein.

In einer alternativen bevorzugten Ausführungsform ist die wenigstens eine Authentifizierungsvorrichtung als mechanische Schlossvorrichtung ausgebildet. Eine derartige Ausführung bietet einen geringeren Kopierschutz als die Verwendung von elektromagnetischen Sensoren und erfordern mechanische Methoden, um einen Infusionsartikel erkennen zu können. Die mechanische Kodierung kann in Form von Erhebungen oder Löchern/Lochstreifen ausgebildet sein, die durch gedrückte Federn/Tastern aktiviert werden und damit die Authentifizierung des Infusionsartikels bewirken.

Die Authentifizierung des Infusionsartikels umfasst bevorzugt eine Erfassung wenigstens eines Authentifizierungsmerkmals, welches am Infusionsartikel angeordnet ist. Das Erfassen kann über einen Sensor erfolgen oder auch mechanisch, beispielsweise durch einen Schlüssel-Schloss-Mechanismus. Bei einem optischen Sensor erfolgt das Erfassen beispielsweise durch Erfassen Scannen eines QR-Codes, insbesondere durch Scannen des QR-Codes. Die Authentifizierung des Infusionsartikels umfasst weiterhin bevorzugt einen Abgleich des Authentifizierungsmerkmals bzw. darin enthaltener Informationen mit einer Referenz, insbesondere einer Datenbank mit Informationen zu Infusionsartikeln, welche für die Verwendung mit der Infusionspumpe zertifiziert sind.

Die Infusionspumpe ist in einer ersten bevorzugten Ausführungsform als Spritzenpumpe ausgebildet. In einer zweiten bevorzugten Ausführungsform ist die Infusionspumpe als Peristaltikpumpe ausgebildet.

In Bezug auf das Infusionssystem wird die oben genannte Aufgabe erfindungsgemäß gelöst durch ein Infusionssystem, umfassend eine oben beschriebene Infusionspumpe und einen Infusionsartikel, wobei der Infusionsartikel wenigstens ein mit der wenigstens einen Authentifizierungsvorrichtung erfassbares und authentifizierbares Authentifizierungsmerkmal aufweist.

Sobald ein Infusionsartikel in die Infusionspumpe eingelegt wird, erkennt der Sensor das auf dem Infusionsartikel aufgebrachte Authentifizierungsmerkmal und liest die zugehörigen Daten aus, das heißt der Sensor erfasst das Authentifizierungsmerkmal. Die auf diese Weise ausgelesenen Daten werden gegenüber der, insbesondere bei Herstellung des Infusionsartikels erstellten, Zertifikats/Gültigkeitstabelle geprüft. Sofern der Datenabgleich von ausgelesenen Daten und den im Zertifikat/der Gültigkeitstabelle abgelegten Daten erfolgreich ist, gilt der Infusionsartikel als authentifiziert und kann in der Infusionspumpe genutzt werden.

Anderenfalls verwehrt die Infusionspumpe dem Nutzer die Anwendung dieses Infusionsartikels, um damit sicherzustellen, dass ausschließlich ein zertifizierter, d.h. vom Hersteller freigegebener, Infusionsartikel genutzt wird. Dies ist insbesondere daher notwendig, um die ausgewiesene Flussrate und deren Toleranz in der Infusionspumpe mit dem Infusionsartikel zu erreichen und damit die Qualität/Güte des Produktes sicherzustellen. Der Datenabgleich kann dabei in der Infusionspumpe erfolgen, er erfolgt aber bevorzugt auf einem Server, der diese Daten, sodass die Gültigkeitstabelle in der Hoheit des Herstellers verbleibt.

Der Infusionsartikel ist beispielsweise ein Infusionsschlauch oder eine Spritze.

Das Authentifizierungsmerkmal umfasst Informationen, die eine eindeutige Zertifizierung bzw. Gültigkeit des Infusionsartikels in der Infusionspumpe erlauben. Dies sind insbesondere Seriennummer / laufende Nummer, Losnummer, Produktionsdatum, Produktionsort, Käufer und/oder Hersteller. Das Authentifizierungsmerkmal ist dabei bevorzugt als QR-Code ausgebildet, der an dem Infusionsartikel angebracht ist, beispielsweise, insbesondere durch Siebdruck oder Laser, aufgedruckt oder aufgeklebt ist.

Alternativ kann das Authentifizierungsmerkmal als RFID-Tag ausgebildet sein, welcher an dem Infusionsartikel angeordnet ist.

Die Infusionspumpe ist vorteilhafterweise ausgebildet, das erfasste Authentifizierungsmerkmal durch Abgleich mit einer Datenbank zu authentifizieren.

Die Datenbank weist bevorzugt eine Zertifikatstabelle bzw. Gültigkeitstabelle auf bzw. ist als eine derartige Tabelle ausgebildet.

Die Datenbank kann dabei in der Infusionspumpe selbst hinterlegt sein oder auf einem Server hinterlegt sein.

In einer ersten bevorzugten Ausführungsform ist die Datenbank in der Infusionspumpe hinterlegt. Dadurch kann eine Authentifizierung direkt von der Infusionspumpe durchgeführt werden. Die Zertifikate/Gültigkeitstabellen werden vor Nutzung der Infusionsartikel durch den Käufer in der Infusionspumpe gespeichert. Wird ein Infusionsartikel in die Infusionspumpe eingelegt und die Authentifizierungsdaten werden vom Infusionseinmalartikel ausgelesen, so erfolgt die Authentifizierung der ausgelesenen Daten gegenüber den im Zertifikat/ Gültigkeitstabelle abgelegten Daten. Wird deren Gültigkeit bestätigt, lässt sich der Infusionsartikel in der Infusionspumpe nutzen, sonst nicht.

In einer zweiten bevorzugten Ausführungsform umfasst das Infusionssystem einen Server, wobei die Datenbank auf dem Server hinterlegt ist, und wobei die Infusionspumpe ausgebildet ist, zu einem erfassten Authentifizierungsmerkmal eine Anfrage an den Server zu richten, und wobei der Server ausgebildet ist, eine Antwort an die Infusionspumpe zu senden, in welcher enthalten ist, ob die in dem Authentifizierungsmerkmal enthaltenen Informationen in der Datenbank enthalten sind.

Eine zentrale Authentifizierung unter Nutzung eines Servers des jeweiligen Infusionsartikels durch die Infusionspumpe ist der lokalen Authentifizierung, bei welcher die Datenbank in der Infusionspumpe hinterlegt ist, vorzuziehen, da sie weniger aufwändig ist, jedoch eine starke Vernetzung und damit eine Kommunikationsmethode innerhalb der Infusionspumpe erfordert. Die Infusionspumpe weist dazu vorteilhafterweise ein Kommunikationsmodul auf, welches ausgebildet ist, zu dem Server einen bidirektionalen Kommunikationskanal für Austausch von Informationen aufzubauen. Das Kommunikationsmodul basiert bevorzugt auf WiFi/WLAN, Mobilfunk oder Satellitenkommunikation. Die Datenbank ist auf diesem Server abgelegt.

Nach Auslesen der Authentifizierungsdaten vom Infusionsartikel baut die Infusionspumpe einen Kommunikationskanal zum Server auf und authentifiziert/verifiziert die ausgelesenen Daten auf ihre Gültigkeit. Handelt es sich um valide Daten, kann der Infusionsartikel uneingeschränkt verwendet werden. Im anderen Fall lehnt die Infusionspumpe den eingelegten Infusionsartikel ab und verweigert dessen Nutzung.

Die Infusionspumpe ist vorteilhafterweise dazu ausgebildet ist, die Nutzung des Infusionsartikels abzulehnen, wenn das Authentifizierungsmerkmal in der Datenbank nicht vorhanden ist, und andernfalls zu erlauben. Der Infusionsartikel ist bevorzugt mit einem QR-Code versehen, in dem bevorzugt eine Losnummer und/oder eine Seriennummer sowie der Hersteller hinterlegt sind. Diese Informationen werden von der Infusionspumpe vorteilhafterweise über einen Sensor, insbesondere eine Kamera, auslesen und dann in Form einer Anfrage an einen Server auf Validität überprüfen. Die Infusionspumpe vergleicht, ob der Infusionsartikel bereits zuvor verwendet wurde (incl. einer gewissen Karenzzeit) und lehnt die Nutzung ab, falls der Infusionsartikel bereits genutzt worden ist.

Die Datenbank enthält bevorzugt verschlüsselte bzw. enkodierte Authentifizierungsdaten. Authentifizierungsvorrichtung der Infusionspumpe ist in diesem Fall dazu ausgebildet, die Korrektheit der aufgespielten/eingespielten Zertifikate/Gültigkeitstabellen zu erkennen, beispielsweise durch Private/Public Keys. Ungültige Zertifikate werden von der Infusionspumpe abgelehnt/nicht verarbeitet.

In Bezug auf das Verfahren wird die oben genannte Aufgabe erfindungsgemäß gelöst, indem in die Infusionspumpe ein Infusionsartikel eingelegt wird und/oder an sie angeschlossen wird, und wobei durch die Infusionspumpe durch Abgleich mit einer Zertifikatsdatenbank überprüft wird, ob der Infusionsartikel für den Betrieb mit dem Infusionsartikel freigegeben ist.

Die Vorteile der Erfindung liegen insbesondere darin, dass durch die einer Inbetriebnahme der Infusionspumpe vorausgehende Authentifizierungsmöglichkeit eines Infusionsartikels eine optimierte Patientenversorgung zuverlässig erfolgen kann und Patientenschäden, die aufgrund der Verwendung von nicht-zertifizierten Artikeln drohen, im Vorhinein verhindert werden.

Ein Ausführungsbeispiel der Erfindung wird anhand einer Zeichnung näher erläutert. Darin zeigen in stark schematisierter Darstellung:
- FIG. 1: eine Peristaltikpumpe mit eingelegtem Patientenversorgungschlauch;
- FIG. 2: eine Spritzenpumpe mit eingelegtem Infusionsschlauch;
- FIG. 3: eine Spritze mit aufgebrachtem QR-Code;
- FIG. 4: einen Infusionsschlauch mit aufgebrachtem QR-Code;
- FIG. 5: eine Infusionspumpe mit eingelegtem Infusionsartikel, und

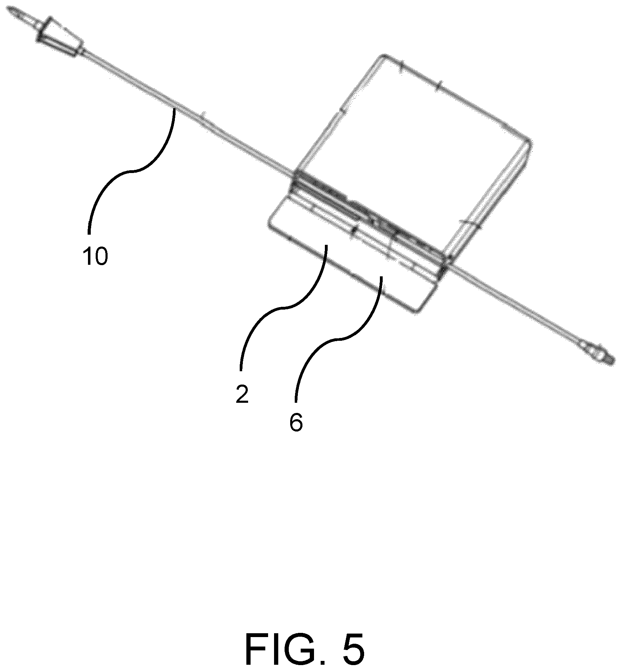

- FIG. 5: eine Abbildung des Datenflusses zur Authentifizierung mit einem Server.

Gleiche Teile sind in allen Figuren mit denselben Bezugszeichen versehen.

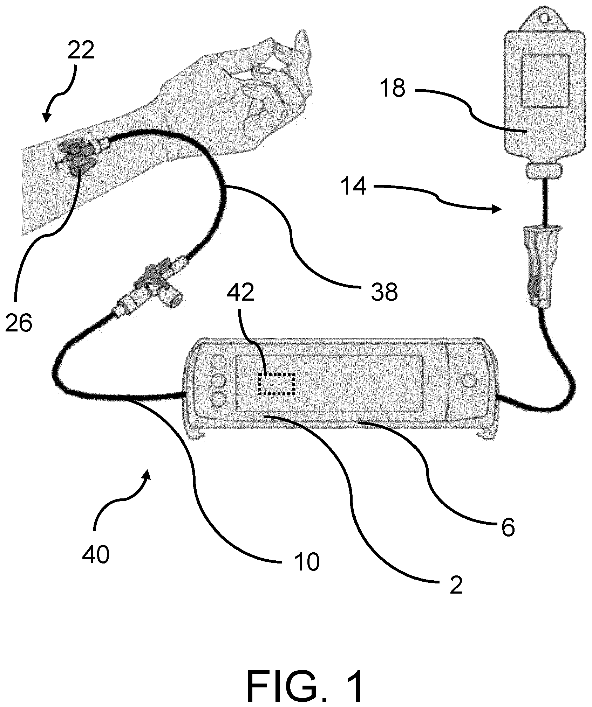

In FIG. 1 ist eine Infusionspumpe 2 dargestellt, welche als Peristaltikpumpe 6 bzw. Schlauchpumpe ausgebildet ist, d.h. als Verdrängerpumpe, bei der das zu fördernde Medium durch äußere mechanische Verformung eines Schlauches durch diesen hindurchgedrückt wird. In die Peristaltikpumpe 6 ist ein Infusionsschlauch 10 eingelegt, welcher an einem ersten Ende 14 mit einem Infusionsbeutel 18 mit Infusionslösung verbunden ist und an einem zweiten Ende 22 mit einer Kanüle 26 verbunden ist. Die Peristaltikpumpe 6 fördert Infusionslösung durch den Infusionsschlauch 10, sodass dem Patienten mit einer vorgegebenen Dosierungsrate Infusionslösung zugeführt wird.

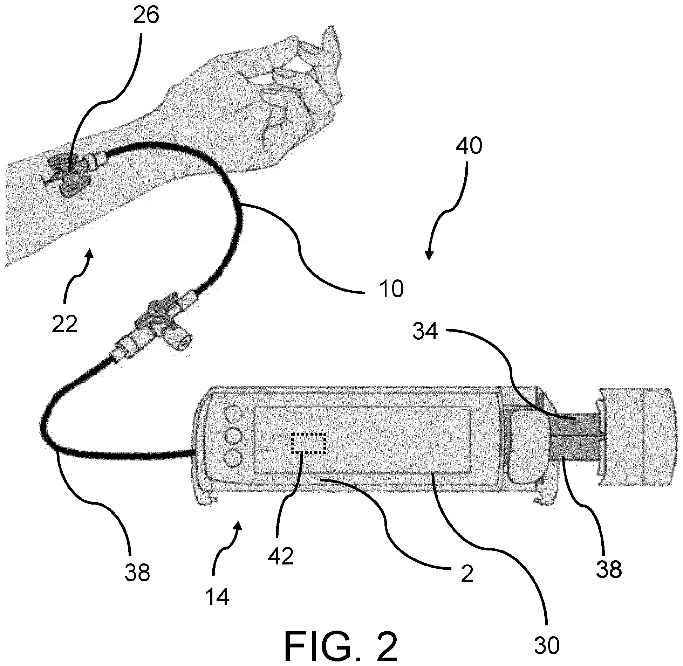

In FIG. 2 ist eine Infusionspumpe 2 dargestellt, die als Spritzenpumpe 30 ausgebildet ist. In die Spritzenpumpe 30 ist eine Spritze 34 eingelegt, welche an einem ersten Ende 14 (in der Spritzenpumpe 30) mit einem Infusionsschlauch 10 verbunden ist und an einem zweiten Ende 22 mit einer Kanüle 26 verbunden ist.

Der Infusionsschlauch 10 bzw. die Spritze 34 sind Infusionsartikel 38, die zusammen mit der Infusionspumpe 2 verwendet werden. In FIG.1 und FIG. 2 bilden Infusionspumpe 2 und Infusionsartikel 38 jeweils ein Infusionssystem 40. Eine zuverlässige Patientenversorgung ist nur möglich, wenn nur ein Infusionsartikel 38 mit der jeweiligen Infusionspumpe 2 verwendet wird, der dafür zertifiziert ist bzw. gültig ist. Nur zertifizierte Infusionsartikel 38 sollten mit der Infusionspumpe 2 verwendet werden; ein Betrieb der Infusionspumpe 2 mit einem nicht zertifizierten Infusionsartikel 38 sollte nicht möglich sein.

Dazu ist vorgesehen, dass die Infusionspumpe 2 eine Authentifizierungsvorrichtung 42 (gestrichelt dargestellt, innerhalb der Infusionspumpe) aufweist, welche den Infusionsartikel 38 authentifiziert. Die Authentifizierungsvorrichtung 42 umfasst einen Sensor 46, welcher vorliegend als Kamera ausgebildet.

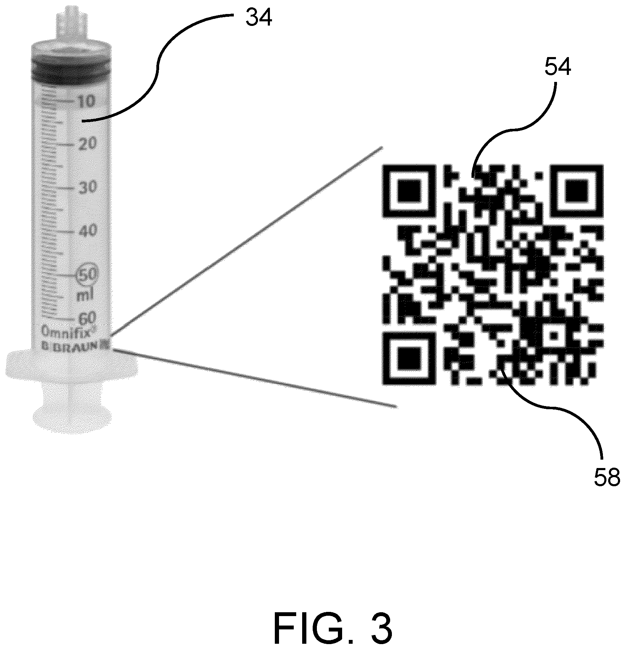

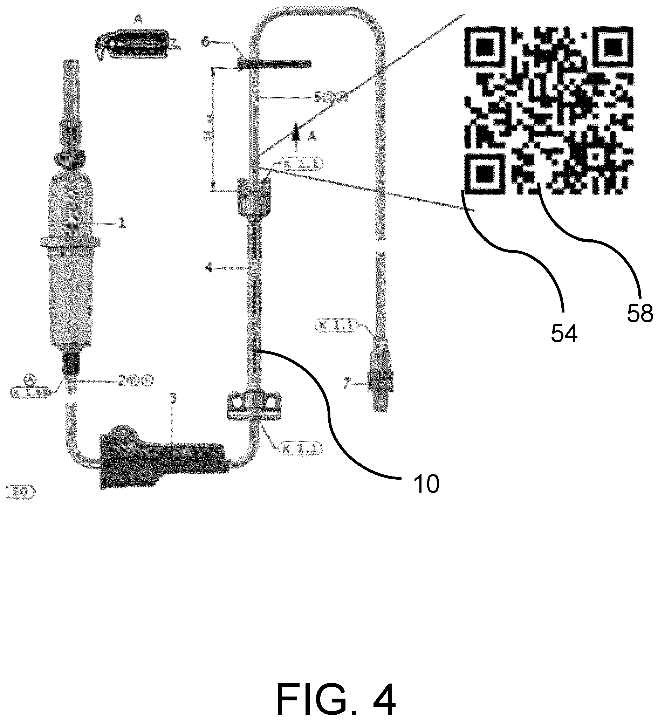

Der Infusionsartikel 38 weist dazu wenigstens ein Authentifizierungsmerkmal 54 auf (siehe FIG. 3 und FIG. 4). Das Authentifizierungsmerkmal 54 wird von der Authentifizierungsvorrichtung 42 authentifiziert, was im Folgenden näher beschrieben wird.

In FIG. 3 ist beispielsgemäß eine Spritze 34 für Infusionslösung dargestellt. Die Spritze 34 weist ein Authentifizierungsmerkmal 54 auf, welches vorliegend als QR-Code 58 ausgebildet ist und rechts in FIG. 3 vergrößert dargestellt ist. In der Spritzenpumpe 30 gemäß FIG. 2 ist eine Kamera (nicht dargestellt) derart angeordnet, dass beim Einlegen der Spritze 34 für den normalen Gebrauch die Kamera dem QR-Code 58 gegenüberliegt, sodass der QR-Code 58 von der Kamera erfasst wird.

In FIG. 4 ist beispielsgemäß ein Patientenversorgungschlauch 10 dargestellt, welcher ein Authentifizierungsmerkmal 54 aufweist, welches als QR-Code 58 ausgebildet ist, der rechts in der FIG. 4 vergrößert dargestellt ist. In FIG. 5 ist eine Peristaltikpumpe 6 mit einem eingelegtem Infusionsschlauch 10 dargestellt.

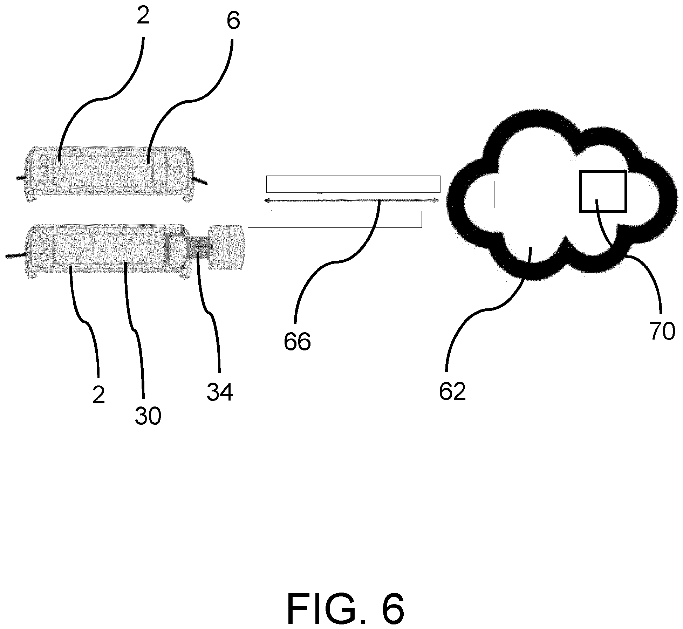

In FIG. 6 sind schematisch eine Peristaltikpumpe 6 und eine Spritzenpumpe 30 und ein Server 62 dargestellt. Durch einen Doppelpfeil 66 ist ein bidirektionaler Informationsaustausch zwischen der jeweiligen Infusionspumpe 2 und dem Server 62 dargestellt. Die Infusionspumpe 2 weist dazu ein Kommunikationsmodul auf zur Kommunikation mit dem Server 62.

Nachdem die Kamera der Authentifizierungsvorrichtung 42 der Infusionspumpe 2 das Authentifizierungsmerkmal 54 an dem Infusionsartikel 38 erfasst hat, extrahiert die Authentifizierungsvorrichtung 42 die darin enthaltenen Informationen und sendet diese bevorzugt verschlüsselt an den Server 62. Der Server 62 gleicht diese Informationen mit den in einer auf dem Server 62 hinterlegten bzw. installierten Datenbank 70 hinterlegten Informationen ab. Sofern die von dem Kommunikationsmodul der Infusionspumpe 2 übermittelten Informationen in der Datenbank 70 hinterlegt bzw. abgelegt sind, signalisiert der Server 62 dies dem Kommunikationsmodul der Infusionspumpe 2 und die Infusionspumpe 2 kann mit diesem Infusionsartikel 38 betrieben werden. Die Infusionspumpe 2 erlaubt in diesem Fall die Verwendung des Infusionsartikels 38. Die Authentifizierung des Infusionsartikels 38 verlief in diesem Fall positiv.

Sofern die oben genannten Informationen in der Datenbank fehlen und/oder nicht vollständig enthalten sind, signalisiert der Server 62 dies dem Kommunikationsmodel der Infusionspumpe 2. Die Authentifizierung des Infusionsartikels 38 verlief in diesem Fall negativ. Die Infusionspumpe 2 blockiert dann den Betrieb mit diesem Infusionsartikel 38. Dies kann beispielsweise dadurch erfolgen, dass die Infusionspumpe 2 nicht pumpt und eine Neueinlage des Infusionsartikels erfordert, ggf. mit einer Aufforderung über eine Anzeige an den Nutzer. Bevorzugt wird dem Nutzer eine Anzeige dargestellt, die das Ablehnen des Infusionsartikels 38 mit Begründung angibt. Zusätzlich werden die Informationen des Ablehnens in der Infusionspumpe 2 protokolliert, ggf. erfolgt zusätzlich feine Protokollierung auch auf dem Server 62. Parallel lässt sich über den Server 62 eine Auswertung der bereits benutzten/verwendeten Infusionsartikel 38 machen und ggf. die erneute Nutzung desselben Infusionsartikels 38 verbieten und/oder kenntlich machen, dass ein Infusionsartikel 38 mehrfach genutzt werden sollte. Weiterhin ist möglich, dass nicht nur eine Anzeige auf der Infusionspumpe 2 hinsichtlich des Grundes der Nichtverfügbarkeit des Infusionsartikels angezeigt wird, sondern auch auf dem Server 62 eine Anzeige einer Fehlermeldung (incl. Protokollierung) erfolgt. Ferner ist denkbar, eine Warnung an den Hersteller zu senden, dass an einer seiner Geräte ein Infusionsartikel 38 genutzt wird/wurde, der nicht authentifiziert ist.

### Bezugszeichenliste

- 2: Infusionspumpe
- 6: Peristaltikpumpe
- 10: Infusionsschlauch
- 14: erstes Ende
- 18: Infusionsbeutel
- 22: zweites Ende
- 26: Kanüle
- 30: Spritzenpumpe
- 34: Spritze
- 38: Infusionsartikel
- 40: Infusionssystem
- 42: Authentifizierungsvorrichtung
- 46: Sensor
- 54: Authentifizierungsmerkmal
- 58: QR-Code
- 62: Server
- 66: Doppelpfeil
- 70: Datenbank

## Patentansprüche

1. Infusionspumpe (2), welche zur Verwendung wenigstens eines Infusionsartikels (38) ausgebildet ist,
**dadurch gekennzeichnet, dass**
die Infusionspumpe (2) wenigstens eine Authentifizierungsvorrichtung (42) zur Authentifizierung des wenigstens einen Infusionsartikels (38) aufweist.

2. Infusionspumpe (2) nach Anspruch 1, wobei die wenigstens eine Authentifizierungsvorrichtung (42) einen Sensor (46) umfasst.

3. Infusionspumpe (2) nach Anspruch 2, wobei der Sensor (46) ein elektromagnetischer Sensor ist.

4. Infusionspumpe (2) nach Anspruch 2, wobei der Sensor (46) als optischer Sensor (46), insbesondere als Kamera, ausgebildet ist.

5. Infusionspumpe (2) nach Anspruch 1, wobei die wenigstens eine Authentifizierungsvorrichtung (42) als mechanische Schlossvorrichtung ausgebildet ist.

6. Infusionspumpe (2) nach einem der Ansprüche 1 bis 5, welche als Spritzenpumpe (30) ausgebildet ist.

7. Infusionspumpe (2) nach einem der Ansprüche 1 bis 5, welche als Peristaltikpumpe (6) ausgebildet ist.

8. Infusionssystem (40), umfassend eine Infusionspumpe (2) nach einem der vorherigen Ansprüche und einen Infusionsartikel (38), wobei der Infusionsartikel (38) wenigstens ein mit der wenigstens einen Authentifizierungsvorrichtung (42) erfassbares und authentifizierbares Authentifizierungsmerkmal (54) aufweist.

9. Infusionssystem (40) nach Anspruch 8, wobei die Infusionspumpe (2) ausgebildet ist, das erfasste Authentifizierungsmerkmal (54) durch Abgleich mit einer Datenbank (70) zu authentifizieren.

10. Infusionssystem (40) nach Anspruch 9, wobei die Datenbank (70) eine Zertifikatstabelle umfasst.

11. Infusionssystem (40) nach Anspruch 10, wobei die Datenbank (70) in der Infusionspumpe (2) hinterlegt ist.

12. Infusionssystem (40) nach Anspruch 10, umfassend einen Server (62), wobei die Datenbank (70) auf dem Server (62) hinterlegt ist, und wobei die Infusionspumpe (2) ausgebildet ist, zu einem erfassten Authentifizierungsmerkmal (54) eine Anfrage an den Server (62) zu richten, und wobei der Server (62) ausgebildet ist, eine Antwort an die Infusionspumpe (2) zu senden, in welcher enthalten ist, ob die in dem Authentifizierungsmerkmal (54) enthaltenen Informationen in der Datenbank (70) enthalten sind.

13. Infusionssystem (40) nach einem der Ansprüche 9 bis 12, wobei die Infusionspumpe (2) ausgebildet ist, die Nutzung des Infusionsartikels (38) abzulehnen, wenn das Authentifizierungsmerkmal (54) in der Datenbank (70) nicht vorhanden ist, und andernfalls zu erlauben.

14. Infusionssystem (40) nach einem der Ansprüche 11 bis 13, wobei die Datenbank (70) verschlüsselte Authentifizierungsdaten enthält.

15. Verfahren zum Betreiben einer Infusionspumpe (2), wobei in die Infusionspumpe (2) ein Infusionsartikel (38) eingelegt wird und/oder an sie angeschlossen wird, und wobei durch die Infusionspumpe (2) durch Abgleich mit einer Zertifikatsdatenbank überprüft wird, ob der Infusionsartikel (38) für den Betrieb mit dem Infusionsartikel (38) freigegeben ist.
